# EUROPÄISCHE PATENTSCHRIFT

(11) **EP 3 360 528 B1**
(45) Veröffentlichungstag und Bekanntmachung des Hinweises auf die Patenterteilung: **11.12.2019**
(21) Anmeldenummer: 18153263.1
(22) Anmeldetag: 24.01.2018
(51) Int. Cl.: A61G 12/00, G16H 40/63, H05K 5/02, H05K 7/14, A61B 5/0402

(54) **MODULARES MEDIZINISCHES INTENSIVTHERAPIEGERÄT**
MODULAR INTENSIVE CARE APPARATUS
APPAREIL MODULAIRE DE SOINS INTENSIFS

(30) Priorität: 08.02.2017 DE 102017102503
(43) Veröffentlichungstag der Anmeldung: 15.08.2018
(73) Patentinhaber: Kagan, Eugen, 53859 Niederkassel Lülsdorf (DE)
(72) Erfinder: Kagan, Eugen, 53859 Niederkassel Lülsdorf (DE)
(74) Vertreter: Wagner Albiger & Partner Patentanwälte mbB

(56) Entgegenhaltungen:
- WO-A1-2017/064318
- US-A1- 2006 221 582

## Beschreibung

Die Erfindung betrifft ein modulares medizinisches Intensivtherapiegerät.

Derartige modulare Geräte umfassend ein Basisgerät mit mehreren Modulsteckplätzen und einer Anzahl lösbar an den Modulsteckplätzen befestigbarer Module sowie eine Steuerungseinrichtung für die befestigten Module, wobei die Modulsteckplätze von Hand betätigbare Verriegelungseinrichtungen für die Module aufweisen. Im Bereich der Intensivtherapie sind derartige modulare Geräte beispielsweise aus der DE 10 2012 103029 A1 bekannt geworden und gestatten die Ausrüstung des die Bedienung und Steuerung aller Module ermöglichenden Basisgeräts mit unterschiedlichen Funktionsmodulen je nach Patientenanforderung. Die Module können mit einfachen Handgriffen schnell und flexibel am Basisgerät angebracht, entfernt und ausgetauscht werden. Ergänzend wird zum Stand der Technik auf die WO 2015 / 094 248 A1 sowie die DE 10 2015 117 581 A1 verwiesen.

Aus der US 2006/0221582 A1 ist ein modulares Gerät mit Modulsteckplätzen für eine Anzahl von Platinen für Telekommunikationszwecke bekannt, bei dem bei Entfernen einer Platine zum Schutz von Daten ein Abschaltvorgang eingeleitet wird.

An die Verriegelung der Module im Bereich der jeweiligen Modulsteckplätze des Basisgeräts werden jedoch insbesondere bei mobilem (Rettungs-) Einsatz eines solchen modularen Geräts hohe mechanische Anforderungen zum Beispiel in Bezug auf die Rüttel- und Schlagfestigkeit gestellt, andererseits muss sichergestellt werden, dass ein für die Erhaltung der Vitalfunktionen eines Patienten notwendiges Modul nicht unbeabsichtigt entfernt wird oder beim Freigeben des Modulsteckplatzes unbeabsichtigt zerstört oder in seiner Funktionsfähigkeit etwa durch abruptes Unterbrechen einer ablaufenden Software eingeschränkt wird. Die Signalübertragung einzelner Kontakte, z.B. in Form von Pins zwischen Basisgerät und den Modulen darf keinesfalls unterbrochen werden und die Befestigung soll klapper- und vibrationsfrei erfolgen und muss leicht ver- und entriegelbar sein. In einem Falltest müssen die Komponenten bruchresistent sein, ferner soll eine Abdichtung gegen eindringende Feuchtigkeit z.B. gemäß IP 54 gewährleistet sein.

Aufgabe der Erfindung ist es daher, ein modulares Gerät der eingangs genannten Art vorzuschlagen, dessen Verriegelungseinrichtungen einfach von Hand bedienbar sind, jedoch eine stabile Modulverriegelung am Modulsteckplatz ermöglichen, gegen Fehlbedienungen geschützt sind und ein vollständiges funktionserhaltendes Abschalten eines Moduls vor dessen Entfernung vom Modulsteckplatz des Basisgeräts sicherstellt.

Diese Aufgabe wird erfindungsgemäß mit einem modularen Gerät gemäß den Merkmalen des Patentanspruchs 1 gelöst. Vorteilhafte Ausgestaltungen und Weiterbildungen der Erfindung sind Gegenstand der abhängigen Ansprüche.

Die Erfindung schlägt vor, dass die Verriegelungseinrichtungen ein um eine Schwenkachse aus einer ersten Endposition über eine Zwischenposition in eine zweite Endposition verschwenkbares handbetätigtes Befestigungsmittel umfassen, welches in der ersten Endposition das Modul verriegelt und in der zweiten Endposition das Modul freigibt. Ferner sind Detektionsmittel zum Erkennen zumindest der ersten Endposition und der Zwischenposition des Betätigungsmittels vorgesehen, wobei das Betätigungsmittel so ausgebildet ist, dass es durch einen Bedienereingriff aus der ersten Endposition zunächst nur in die Zwischenposition und von dort über einen zweiten Bedienereingriff weiter in die zweite Endposition verschwenkbar ist. Das Verlagern des Betätigungsmittels aus der das Modul verriegelnden ersten Endposition in die das Modul freigebende zweite Endposition kann damit nicht in einem einzigen Zug über einen Bedienereingriff erfolgen, sondern nach Erreichen der das Modul noch immer verriegelnden Zwischenposition muss ein zweiter Bedienereingriff bewusst erfolgen, um letztlich die Freigabe durch Erreichen der zweiten Endposition zu erreichen. Hierdurch wird einem versehentlichen Freigeben des Moduls wirksam vorgebeugt. Überdies ist erfindungsgemäß vorgesehen, dass die Detektionsmittel mit der Steuerungseinrichtung kommunizieren, dergestalt, dass beim Verschwenken des Betätigungsmittels aus der ersten Endposition in Richtung der Zwischenposition von der Steuerungseinrichtung ein Alarm ausgebbar ist und bei weiterem Verschwenken des Betätigungsmittels in Richtung der zweiten Endposition das Modul von der Steuerungseinrichtung abschaltbar ist. Der Alarm kann lediglich bei aktivem, eingeschaltetem Modul erfolgen, andernfalls kann eine entsprechende Warnmeldung ausgegeben werden, wenn das Modul inaktiv ist.

Ein Bediener muss von daher nach dem ersten Bedienereingriff und Erreichen der Zwischenposition den ausgelösten Alarm bewusst quittieren, indem er den zweiten Bedienereingriff in Richtung der zweiten Endposition ausführt. In dieser Zeit kann sodann die Steuerungseinrichtung das vom Bedienter willentlich freigegebene Modul ordnungsgemäß abschalten, beispielsweise die Software herunterfahren, einen Abmeldevorgang durchführen und die Stromversorgung abschalten.

Sollte jedoch der erste Bedienereingriff bis zur Zwischenposition versehentlich erfolgt sein, wird der Bediener auch angesichts des unmittelbar ausgelösten Alarms, der beispielsweise optisch und/oder akustisch erfolgen kann, den erforderlichen zweiten Bedienereingriff nicht mehr ausführen, sondern das Betätigungsmittel wieder in die erste Endposition zurückführen, woraufhin der Alarm erlischt. Da in dieser gesamten Zeit der Bewegung des Betätigungsmittels aus der ersten Endposition in die Zwischenposition und wieder zurück in die erste Endposition das Modul zu keinem Zeitpunkt von dem Modulsteckplatz am Basisgerät freigegeben wurde und auch keine Abschaltung desselben erfolgte, bleibt die u.U. für einen Patienten lebenswichtige Funktion des betreffenden Moduls ununterbrochen erhalten. Das erfindungsgemäße modulare Gerät erreicht von daher eine äußerst hohe Funktionssicherheit auch gegen Fehlbedienung, die den Anforderungen an ein medizinisches Gerät Rechnung trägt. Andererseits kann aber durch entsprechende Gestaltung des Betätigungsmittels eine einfache Verriegelung und Freigabe der Module am und vom Basisgerät ermöglicht werden.

Erfindungsgemäß ist vorgesehen, dass das Betätigungsmittel von einer um die Schwenkachse drehbar gelagerten Scheibe gebildet ist, die zwei durch einen Steg voneinander getrennte radial nach innen verlaufende Eingriffsausnehmungen für einen Finger einer Bedienperson aufweist und das Betätigungsmittel derart benachbart zu einem Modulsteckplatz angeordnet ist, dass eine erste Eingriffsausnehmung in der ersten Endposition von außen zugänglich ist und ein Verschwenken mittels Fingereingriff in diese erste Eingriffsausnehmung bis zur Zwischenposition ermöglicht ist, während die zweite Eingriffsausnehmung verdeckt ist und in der Zwischenposition die zweite Eingriffsausnehmung von außen zugänglich ist und mittels Fingereingriff in die zweite Eingriffsausnehmung ein Verschwenken der Scheibe bis zur zweiten Endposition ermöglicht ist.

Durch diese Ausgestaltung wird einem Bediener eine zwangsweise Abfolge von Bedienungsschritten, nämlich Fingereingriff in die erste Eingriffsausnehmung und Verschwenken der Scheibe aus der ersten Endposition bis in die Zwischenposition unter Auslösung eines Alarms und nachfolgend weiterer Fingereingriff in die erst in der Zwischenposition zugängliche zweite Eingriffsausnehmung, welche ein Verschwenken bis in die zweite Endposition gestattet, vorgegeben. Eine solche Ausführung mehrerer Schritte während eines ausgelösten Alarms kann zuverlässig nicht mehr versehentlich erfolgen, gestaltet sich jedoch so einfach, dass sie intuitiv auch unter Stressbedingungen durchgeführt werden kann.

Nach einem weiteren Vorschlag der Erfindung umfassen die Verriegelungseinrichtungen eine Verriegelungsausnehmung im Bereich der Modulsteckplätze des Basisgeräts und die Module einen in die Verriegelungsausnehmung einführbaren vorstehenden Haken, wobei das Betätigungsmittel so geformt ist, dass es außerhalb der zweiten Endposition den vorstehenden Haken des Moduls in der Verriegelungsausnehmung verriegelnd hintergreift. Insoweit dient das Betätigungsmittel nicht nur zum Durchführen der erforderlichen Handgriffe zum Verriegeln bzw. Freigeben eines Moduls, sondern stellt auch das eigentliche Verriegelungs- bzw. Freigabeorgan für die Module dar, wodurch der bauliche Aufwand und die Teilevielfalt bedeutend verringert werden.

Die im Rahmen der Erfindung vorgesehenen Detektionsmittel können beispielsweise magnetische, optische oder elektrische Sensoren und/oder Kontakte umfassen, wobei insbesondere Hallsensoren als magnetische Detektionsmittel vorgesehen sind, die mit Magnetelementen zusammenwirken, mit welchen das Betätigungsmittel ausgerüstet ist, sodass sie mittels der Hallsensoren detektierbar sind und entsprechend eine Detektion der jeweiligen Position des Betätigungsmittels und davon abhängige Auslösung von Alarm- und Abschaltfunktionen der Steuerungseinrichtung für das betreffende Modul bewirken könne.

Nach einer möglichen Ausgestaltung der Erfindung ist dem Betätigungsmittel zugeordnet und gemeinsam mit diesem um die Schwenkachse verschwenkbar eine Schaltfahne vorgesehen, die die Magnetelemente in der für die Funktion erforderlichen eindeutigen Orientierung und Anordnung trägt.

Zur weiteren Steigerung der Funktionssicherheit und Fehlbedienungsresistenz des erfindungsgemäßen modularen Geräts wird vorgeschlagen, dass das Betätigungsmittel in der ersten und zweiten Endposition sowie der Zwischenposition jeweils in dieser lösbar einrastet und zwischen der ersten Endposition und der Zwischenposition in Richtung auf die erste Endposition vorgespannt ist und zwischen der Zwischenposition und der zweiten Endposition in Richtung auf die Zwischenposition vorgespannt ist. Im Falle einer Fehl- bzw. versehentlichen Betätigung des Betätigungsmittels kehrt dieses stets selbsttätig in einen die Funktion des Moduls aufrechterhaltenden Zustand zurück und es wird darüber hinaus eine taktile Rückmeldung für den Bediener erzeugt.

Weitere Ausgestaltungen und Einzelheiten der Erfindung werden nachfolgend anhand der ein Ausführungsbeispiel darstellenden Zeichnungen erläutert. Es zeigen:
- Figur 1: in perspektivischer Darstellung ein modulares Gerät gemäß der Erfindung;
- Figur 2: die Aufsicht auf ein Modul gemäß Figur 1;
- Figur 3: eine Prinzipdarstellung des Betätigungsmittels gemäß der Erfindung in einer ersten Endposition;
- Figur 4: eine Prinzipdarstellung des Betätigungsmittels gemäß der Erfindung in einer Zwischenposition;
- Figur 5: eine Prinzipdarstellung des Betätigungsmittels gemäß der Erfindung in einer zweiten Endposition.

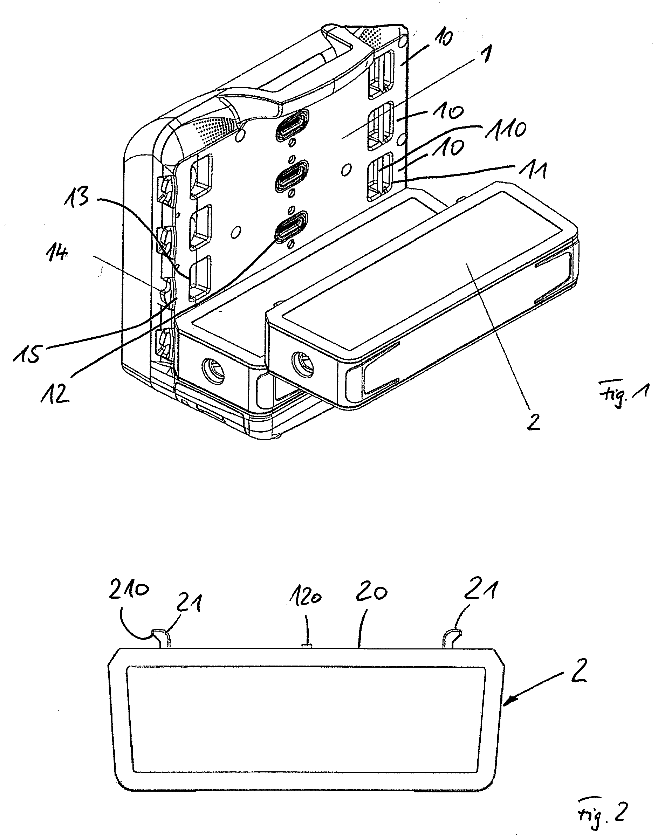

Aus der Figur 1 ist ein modulares Gerät in Form eines modularen Intensivtherapiegerätes zur stationären und mobilen Versorgung eines Patienten dargestellt. Es umfasst ein mit Bezugszeichen 1 gekennzeichnetes Basisgerät mit einer Vielzahl von übereinander angeordneten und gleich ausgebildeten Modulsteckplätzen 10, an denen in nachfolgend noch näher beschriebener Weise einzelne Module 2 lösbar befestigt werden können, um dem Basisgerät 1 weitere Funktionen, die im jeweiligen Anwendungsfall zur Behandlung des Patienten benötigt werden, hinzuzufügen. Beispiele derartiger Funktionen, die in einzelnen Modulen 2 integriert sein können, umfassen Beatmung, EKG-Diagnostik sowie eine Vielzahl weiterer intensivmedizinischer Funktionen, die keiner generellen Beschränkung unterliegen.

Das Basisgerät 1 umfasst dabei nicht näher dargestellter Weise nicht nur eine Energieversorgung für den mobilen Betrieb sowohl des Basisgeräts 1 als auch der angeschlossenen Module 2, sondern auch eine Steuerungseinrichtung, mit welcher die angeschlossenen Module in der gewünschten Weise gesteuert werden können und von den Modulen gelieferte Daten, zum Beispiel Patientenvitaldaten verarbeitet und dargestellt werden können. Auf der in Figur 1 nicht sichtbaren Vorderseite des Basisgeräts 1 ist dazu ein Touch-Screen Monitor vorgesehen.

Jeder Modulsteckplatz 10 verfügt etwa im mittleren Bereich über ein elektrisches Anschlussfeld 12 für die elektrische Kontaktierung des am Modulsteckplatz 10 befestigbaren Moduls 2, wobei selbstverständlich jedes Modul 2 mit einem hierzu korrespondierenden Kontaktelement 120 auf der dem Modulsteckplatz 10 zugewandten Seite 20 ausgerüstet ist. Darüber hinaus ist in der Darstellung gemäß Figur 1 auf der rechten Seite eines jeden Modulsteckplatzes 10 eine Schließausnehmung 11 vorgesehen, in der ein vertikal verlaufender Schließsteg 110 angeordnet ist. Entsprechend ist auf der in Figur 1 linken Seite jedes Modulsteckplatzes 10 eine weitere Verriegelungsausnehmung 13 im Basisgerät 1 vorgesehen, in welche ein nachfolgend noch näher erläutertes Betätigungsmittel 14, welches über Mulden 15 in der Schmalseite des Basisgeräts 1 von außen zugänglich ist, je nach der Positionierung des Betätigungsmittels 14 eingreift oder die Verriegelungsausnehmung 13 freigibt. Jedes Modul ist hierzu korrespondierend auf der dem Modulsteckplatz zugewandten Seite 20 mit vorstehenden Haken 21 versehen, die in die Schließ- und Verriegelungsausnehmung 13 eingreifen und den Steg 110 sowie das in die Verriegelungsausnehmung 13 hineinragende Betätigungsmittel 14 hintergreifen. Hierdurch ist eine sichere Befestigung des Moduls 2 an einem Modulsteckplatz 10 bewirkt, wobei aufgrund der gewählten Konfiguration jedes Modul 2 auch in einer um 180° gedrehten Orientierung angedockt werden kann, etwa um seitliche Anschlussterminals je nach Zugänglichkeit auf die rechte oder linke Seite des modularen Geräts zu verlegen.

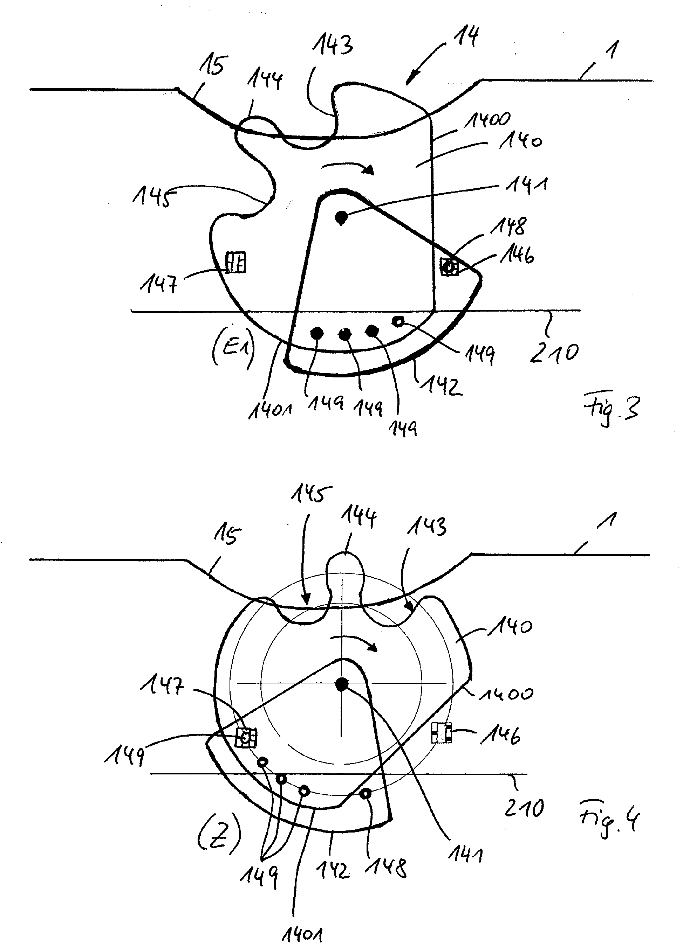

Der Aufbau und die Wirkungsweise des Betätigungsmittels 14 wird in näheren Einzelheiten aus den schematischen Darstellungen gemäß Figuren 3-5 ersichtlich, die das Betätigungsmittel im Zusammenwirken mit einem am Modulsteckplatz 10 befestigten Modul 2 in verschiedenen Positionen und Zuständen zeigen.

Aus der Figur 3 ist das Betätigungsmittel 14 in einer ersten Endposition E1 ersichtlich, in der das Befestigungsmittel 14 das angedockte Modul 2 im Bereich des in die Verriegelungsausnehmung 13 eingreifenden Hakens 21 fest verriegelt, was somit gewissermaßen den Betriebs- und Normalzustand eines am Basisgerät 1 angedockten Moduls 2 darstellt. Es versteht sich, dass dabei der gegenüberliegende Haken 21 den Steg 110 innerhalb der Schließausnehmung 11 ebenfalls hintergreift.

Man erkennt aus der Darstellung gemäß Figur 1, dass das Betätigungsmittel 14 aus der Grundform einer Scheibe 140 herausgearbeitet ist, die in nachfolgend noch näher erläuterter Weise über die seitliche Fingermulde 15 am Basisgerät 1 von außen so zugänglich ist, dass sie zum Beispiel von einem Finger einer nicht dargestellten Bedienperson betätigt werden kann. Die Scheibe 140 ist um eine hier horizontal verlaufende Schwenkachse 141 in der durch Pfeil dargestellten Richtung schwenkbar, wozu die Scheibe 140 mit einer ersten, radial nach innen eingearbeiteten Eingriffsausnehmung 143 versehen ist, die in der dargestellten ersten Endposition E1 über die Fingermulde 15 von außen zugänglich ist. Ein Steg 144 teilt demgegenüber eine zweite Eingriffsausnehmung 145 ab, die jedoch in der dargestellten ersten Endposition E1 innerhalb des Gehäuses des Basisgeräts 1 zum Liegen kommt und von daher über die Fingermulde 15 von außen nicht zugänglich ist.

Auf ihrer der Eingriffsausnehmung 143 in Bezug auf die Schwenkachse 141 radial gegenüberliegenden Seite ist ein mit Bezugszeichen 1401 bezeichneter Bereich der Scheibe 140 so positioniert, dass er das dargestellte freie Ende 210 des Hakens 21 hintergreift und diesen somit in der bereits beschriebenen Weise am Modulsteckplatz 10 verriegelt.

Mit der Scheibe 140 starr verbunden und insoweit gleichermaßen um die Schwenkachse 141 schwenkbar ist eine Schaltfahne 142 vorgesehen, die vier etwa in einem gleichen Abstand zueinander und auf einem gemeinsamen Kreisabschnitt um die Schwenkachse 141 angeordnete Magneten 149 trägt sowie in einem etwas größeren Abstand einen weiteren Magneten, der mit Bezugszeichen 148 versehen ist. Ferner sind in dem von der verschwenkbaren Scheibe 140 und der Schaltfahne 142 bei einer Schwenkbewegung überstreichbaren Bereich 2 Detektionsmittel in Form von Hallsensoren 146,147 ortsfest angeordnet, die so positioniert sind, dass in der dargestellten ersten Endposition E1 der Magnet 148 auf der Schaltfahne 142 über dem Hallsensor 146 positioniert und von daher von diesem erfassbar ist, wohingegen die weiteren Magneten 149 weder vom Hallsensor 146 noch vom weiteren Hallsensor 147 detektiert und erfasst werden, da sie einen entsprechenden Abstand zu diesen aufweisen. Wenn demgemäß der Hallsensor 146 einen Magneten, hier den Magnet 148 detektiert, ist dieser Zustand eindeutig der Positionierung des Betätigungsmittels 14 in der ersten Endposition E1 gemäß Figur 3 zugeordnet und kann der Steuerungseinrichtung als Normal-und Betriebszustand mit verriegeltem Modul 2 entsprechend signalisiert werden, in welchem das Modul 2 bestimmungsgemäß verwendet werden kann.

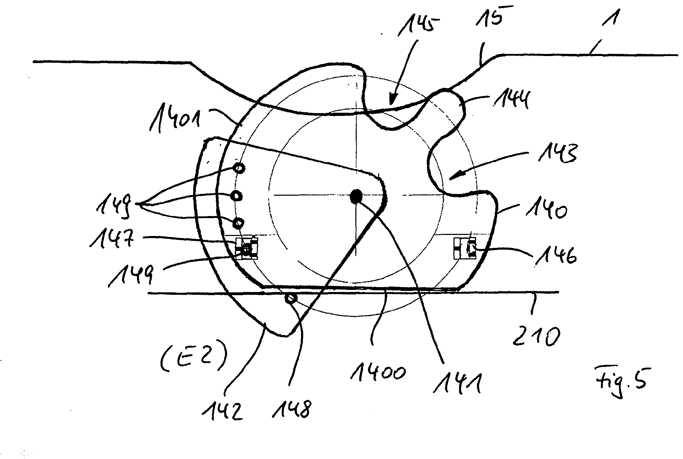

In den Figuren 4 und 5 wird sukzessive aufeinanderfolgend dargestellt, wie ein solches gemäß Figur 3 verriegeltes Modul 2 vom Basisgerät 1 gelöst und nach Freigabe abgenommen werden kann.

Dazu wird zunächst, wie schon anhand der Figur 3 erläutert, mittels eines Fingereingriffs in die erste Eingriffsausnehmung 143 die als Betätigungsmittel 14 dienende Scheibe 140 gemäß der Pfeildarstellung in Figur 3 im Uhrzeigersinn um die Schwenkachse 141 verschwenkt, bis sie die in der Figur 4 dargestellte Zwischenposition Z einnimmt, in der die erste Eingriffsausnehmung 143 im Gehäuse des Basisgeräts 1 verschwindet und nachfolgend nicht mehr für die weitere Betätigung zur Verfügung steht.

In dieser dargestellten Zwischenposition Z verriegelt der mit Bezugszeichen 1401 versehene Abschnitt der Scheibe 140 nach wie vor das freie Ende 210 des Hakens 21, jedoch befindet sich der auf der Schaltfahne 142 positionierte Magnet 148 nicht mehr oberhalb des Hallsensors 146 und wird infolgedessen von diesem auch nicht mehr detektiert. Als unmittelbare Folge gibt der Hallsensor 146 ein entsprechendes Signal an die Steuerungseinrichtung aus, die ihrerseits einen optischen und/oder akustischen Alarm ausgibt. Darüber hinaus gelangt der in der dargestellten Schwenkrichtung 1. der hier vier Magneten 149 in den Erfassungsbereich des gegenüberliegenden weiteren Hallsensors 147, was ebenfalls an die Steuerungseinrichtung signalisiert wird. Neben dem ausgelösten Alarm durch den Hallsensoren 146 kann die Steuerungseinrichtung somit durch den Hallsensor 147 ausgelöst ein Abschalten des noch verriegelten Moduls 2 einleiten, beispielsweise durch Herunterfahren der dort ablaufenden Software.

Dem Bediener wird das Verschwenken in Richtung der Zwischenposition Z durch Ausgabe des Alarms vom Hallsensor 146 signalisiert. Der Bediener kann nun überprüfen, ob der Abschaltung bewusst und gewollt oder versehentlich eingeleitet wurde. Sollte der Schaltvorgang versehentlich eingeleitet worden sein, kann das Betätigungsmittel 14 unmittelbar zurück in die in der Figur 3 dargestellte erste Endposition bewegt werden, in der der Alarm erlischt und der Normalzustand für das Modul 2 herbeigeführt wird, ohne dass die Verriegelung desselben zu irgendeinem Zeitpunkt unterbrochen worden wäre.

Ist jedoch das Verschwenken in Richtung der Zwischenposition Z und die Ausgabe des Alarms auf eine gewollte Betätigung des Dieners zurückzuführen, ist dieser zum Freigeben des bereits im Abschaltvorgang befindlichen Moduls 2 gezwungen, den verwendeten Finger aus der ersten Eingriffsausnehmung 143 bewusst herauszuheben und hinter dem Steg 144 in die nun zugängliche zweite Eingriffsausnehmung 145 einzuführen, um die Scheibe 140 weiter in Schwenkrichtung gemäß dem dargestellten Pfeil in die aus der Figur 5 ersichtliche zweite Endposition E2 zu überführen.

In dieser in der Figur 5 dargestellten zweiten Endposition E2 erfasst der Hallsensor 147 nach wie vor einen der hier vier in gleichen Abstand zueinander angeordneten Magneten 149, wobei der Abstand zwischen den benachbarten Magneten 149 so gewählt ist, dass sie während der gesamten Verschwenkbewegung der Scheibe 140 von der Zwischenposition Z gemäß Figur 4 in die zweite Endposition E2 gemäß Figur 5 stets vom Hallsensor 147 kontinuierlich über dessen Hysteresebereich erfasst werden. Insoweit könnte anstelle mehrerer in gleichem Abstand zueinander angeordneter einzelner Magneten 149 auch ein entsprechender durchgängiger Magnet in diesem Bereich der Schaltfahne 142 vorgesehen sein. Die fortlaufende Erfassung eines Magneten mit dem Hallsensoren 147 stellt sicher, dass das Modul 2 vollständig abgeschaltet wird, bevor die in der Figur 5 dargestellte zweite Endposition der Scheibe 140 erreicht ist.

Man erkennt ferner, dass anschließend an den Abschnitt 1401 der Scheibe 140 ein Scheibensegment entlang einer geraden Freigabekante 1400 ausgespart worden ist, die in der zweiten Endposition E2 parallel zum freien Ende 210 des vorstehenden Hakens 21 des Moduls 2 verläuft, diesen jedoch nicht mehr hintergreift, sodass der Haken 21 des Moduls 2 in dieser dargestellten zweiten Endposition E2 freigegeben und das Modul 2 vom Basisgerät 1 abgenommen werden kann.

Das Befestigen und Andocken eines Moduls 2 an einem der Modulsteckplätze 10 erfolgt dementsprechend in umgekehrter Reihenfolge.

Zur weiteren Erhöhung der Fehlersicherheit und zur Erzeugung einer taktilen Rückmeldung ist darüber hinaus vorgesehen, dass die als Betätigungsmittel 14 dienende Scheibe in den Endpositionen E1 und E2 sowie der Zwischenposition Z lösbar einrastet und zwischen der Endposition E1 und der Zwischenposition Z in Richtung auf die Endposition E1 vorgespannt ist. Gleichermaßen kann auch zwischen der Zwischenposition Z und der zweiten Endposition E2 eine Vorspannung in Richtung auf die Zwischenposition Z vorgesehen sein.

Das vorangehend erläuterte modulare Gerät erreicht durch den dargestellten Aufbau des Betätigungsmittels 14 eine besonders einfache, fehlersichere und fehlbedienungssichere Verriegelung und Freigabe der am Basisgerät 1 wahlweise befestigbaren Module 2, die den Anforderungen an ein medizinisches Gerät mit lebenserhaltender Funktionalität gerecht werden.

## Patentansprüche

1. Modulares medizinisches Intensivtherapiegerät, umfassend ein Basisgerät (1) mit mehreren Modulsteckplätzen (10) und einer Anzahl lösbar an den Modulsteckplätzen (10) befestigbarer Module (2) sowie eine Steuerungseinrichtung für die befestigten Module (2), wobei die Modulsteckplätze (10) von Hand betätigbare Verriegelungseinrichtungen für die Module (2) aufweisen, wobei die Verriegelungseinrichtungen ein um eine Schwenkachse (141) aus einer ersten Endposition (E1) über eine Zwischenposition (Z) in eine zweite Endposition (E2) verschwenkbares handbetätigtes Betätigungsmittel umfassen, welches in der ersten Endposition (E1) das Modul (2) verriegelt und in der zweiten Endposition (E2) das Modul (2) freigibt und Detektionsmittel zum Erkennen zumindest der ersten Endposition (E1) des Betätigungsmittels vorgesehen sind,
**dadurch gekennzeichnet, dass**
zudem Detektionsmittel zum Erkennen der Zwischenposition (Z) des Betätigungsmittels vorgesehen sind und das Betätigungsmittel so ausgebildet ist, dass es durch einen Bedienereingriff aus der ersten Endposition (E1) zunächst nur in die Zwischenposition (Z) und von dort nicht in einem einzigen Zug, sondern über einen zweiten Bedienereingriff weiter in die zweite Endposition (E2) verschwenkbar ist und die Detektionsmittel mit der Steuerungseinrichtung kommunizieren, dergestalt, dass beim Verschwenken des Betätigungsmittels aus der ersten Endposition (E1) in Richtung der Zwischenposition (Z) von der Steuerungseinrichtung ein Alarm ausgebbar ist und bei weiterem Verschwenken des Betätigungsmittels in Richtung der zweiten Endposition (E2) das Modul (2) von der Steuerungseinrichtung abschaltbar ist, wobei das Betätigungsmittel von einer um die Schwenkachse (141) drehbar gelagerten Scheibe (140) gebildet ist, die zwei durch einen Steg (144) voneinander getrennte radial nach innen verlaufende Eingriffsausnehmungen (143, 145) für einen Finger aufweist und das Betätigungsmittel derart benachbart zu einem Modulsteckplatz (10) angeordnet ist, dass eine erste Eingriffsausnehmung (143) in der ersten Endposition (E1) von außen zugänglich ist und ein Verschwenken mittels Fingereingriff in die erste Eingriffsausnehmung (143) bis zur Zwischenposition (Z) ermöglicht ist, während die zweite Eingriffsausnehmung (145) verdeckt ist und in der Zwischenposition (Z) die zweite Eingriffsausnehmung (145) von außen zugänglich ist und mittels Fingereingriff in die zweite Eingriffsausnehmung (145) ein Verschwenken der Scheibe (140) bis zur zweiten Endposition (E2) ermöglicht ist.

2. Modulares Gerät nach Anspruch 1, **dadurch gekennzeichnet, dass** die Verriegelungseinrichtungen eine Verriegelungsausnehmung (13) im Bereich der Modulsteckplätze (10) des Basisgeräts (1) und die Module (2) einen in die Verriegelungsausnehmung (13) einführbaren vorstehenden Haken (21) aufweisen und das Betätigungsmittel so geformt ist, dass es außerhalb der zweiten Endposition (E2) den vorstehenden Haken (21) des Moduls (2) in der Verriegelungsausnehmung (13) verriegelnd hintergreift.

3. Modulares Gerät nach einem der Ansprüche 1 und 2, **dadurch gekennzeichnet, dass** die Detektionsmittel magnetische Sensoren umfassen.

4. Modulares Gerät nach Anspruch 3, **dadurch gekennzeichnet, dass** als magnetische Detektionsmittel Hallsensoren (146, 147) vorgesehen sind und das Betätigungsmittel mehrere Magnetelemente (148, 149) aufweist, die mittels der Hallsensoren (146, 147) detektierbar sind.

5. Modulares Gerät nach Anspruch 4, **dadurch gekennzeichnet, dass** dem Betätigungsmittel zugeordnet und gemeinsam mit diesem um die Schwenkachse (141) verschwenkbar eine Schaltfahne (142) vorgesehen ist, die die Magnetelemente (148, 149) trägt

6. Modulares Gerät nach einem der Ansprüche 1 oder 2, **dadurch gekennzeichnet, dass** die Detektionsmittel optische Sensoren umfassen.

7. Modulares Gerät nach einem der Ansprüche 1 bis 6, **dadurch gekennzeichnet, dass** das Betätigungsmittel in der ersten und zweiten Endposition (E1, E2) sowie der Zwischenposition (Z) jeweils in dieser lösbar einrastet und zwischen der ersten Endposition (E1) und der Zwischenposition (Z) in Richtung auf die erste Endposition (E1) vorgespannt ist und zwischen der Zwischenposition (Z) und der zweiten Endposition (E2) in Richtung auf die Zwischenposition (Z) vorgespannt ist.

8. Modulares Gerät nach einem der Ansprüche 1 bis 7, **dadurch gekennzeichnet, dass** mittels der Steuerungseinrichtung vor dem Abschalten eines Moduls (2) eine auf dem Modul (2) laufende Software herunterfahrbar ist.

## Claims

1. Modular intensive care apparatus comprising a basic device (1) having several module slots (10) and a number of modules (2) releasably fastenable at the module slots (10) and a control device for the fastened modules (2), wherein the module slots (10) have locking means, which can be operated by hand, for the modules (2), wherein the locking means comprise a hand-operated actuating means which can be swivelled about a swivel axis (141) from a first end position (E1) via an interim position (Z) into a second end position (E2), which actuating means locks the module (2) in the first end position (E1) and releases the module (2) in the second end position (E2) and detecting means are provided to detect at least the first end position (E1) of the actuating means,
**characterised in that**
in addition detecting means are provided for detecting the interim position (Z) of the actuating means and the actuating means is designed such that it can be swivelled by means of an operator intervention out of the first end position (E1) initially only into the interim position (Z) and from there not in a single movement but rather by means of a second operator intervention further into the second end position (E2) and the detecting means communicate with the control device in such a manner that when the actuating means is swivelled out of the first end position (E1) in the direction of the interim position (Z) an alarm can be outputted by the control device and when the actuating means is further swivelled in the direction of the second end position (E2) the module (2) can be switched off by the control device, wherein the actuating means is formed by a disc (140) mounted rotatably about the swivel axis (141), which disc has two engaging recesses (143, 145), extending radially to the inside separated from one another by a web (144), for a finger and the actuating means is disposed adjacent to a module slot (10) in such a manner that a first engaging recess (143) is accessible from the outside in the first end position (E1) and a swivelling by means of finger engagement into the first engaging recess (143) is facilitated up until the interim position (Z) while the second engaging recess (145) is covered and in the interim position (Z) the second engaging recess (145) is accessible from the outside and by means of finger engagement into the second engaging recess (145) a swivelling of the disc (140) is facilitated up until the second end position (E2).

2. Modular device according to claim 1, **characterised in that** the locking means have a locking recess (13) in the region of the module slots (10) of the basic device (1) and the modules (2) have a protruding hook (21) which can be introduced into the locking recess (13) and the actuating means are formed such that, outside the second end position (E2) it grips in a locking manner behind the protruding hook (21) of the module (2) in the locking recess (13).

3. Modular device according to any of claims 1 and 2, **characterised in that** the detecting means comprise magnetic sensors.

4. Modular device according to claim 3, **characterised in that** as magnetic detecting means Hall sensors (146, 147) are provided and the actuating means has several magnet elements (148, 149) which can be detected by means of the Hall sensors (146, 147).

5. Modular device according to claim 4, **characterised in that** a switching vane (142), which carries the magnet elements (148, 149), is provided and assigned to the actuating means and can be swivelled together therewith about the swivel axis (141).

6. Modular device according to any of claims 1 or 2, **characterised in that** the detecting means comprise optical sensors.

7. Modular device according to any of claims 1 to 6, **characterised in that** the actuating means in the first and second end position (E1, E2) and the interim position (Z) in each case latches releasably in said position and is pretensioned between the first end position (E1) and the interim position (Z) in the direction towards the first end position (E1) and between the interim position (Z) and the second position (E2) is pretensioned in the direction towards the interim position (Z).

8. Modular device according to any of claims 1 to 7, **characterised in that** by means of the control device, prior to the switching off of a module (2), a software running on the module (2) can be powered down.

## Revendications

1. Appareil de soins intensifs médical modulaire, comprenant un appareil de base (1) avec plusieurs emplacements d'enfichage de module (10) et un nombre de modules (2) pouvant être fixés de manière amovible aux emplacements d'enfichage de module (10) ainsi qu'un dispositif de commande pour les modules (2) fixés, dans lequel les emplacements d'enfichage de module (10) présentent des dispositifs de verrouillage actionnables à la main pour les modules (2), dans lequel les dispositifs de verrouillage comprennent un moyen d'actionnement actionné à la main pivotant autour d'un axe de pivotement (141) d'une première position d'extrémité (E1) en passant par une position intermédiaire (Z) à une seconde position d'extrémité (E2), lequel verrouille le module (2) dans la première position d'extrémité (E1) et libère le module (2) dans la seconde position d'extrémité (E2) et des moyens de détection sont prévus pour la détection d'au moins la première position d'extrémité (E1) du moyen d'actionnement,
**caractérisé en ce que**
en plus des moyens de détection pour la détection de la position intermédiaire (Z) du moyen d'actionnement sont prévus et le moyen d'actionnement est réalisé de sorte qu'il soit pivotant par une intervention d'opérateur de la première position d'extrémité (E1) d'abord seulement à la position intermédiaire (Z) et de là pas d'un seul coup, mais par le biais d'une seconde intervention d'opérateur, à la seconde position d'extrémité (E2) et les moyens de détection communiquent avec le dispositif de commande de sorte que, lors du pivotement du moyen d'actionnement de la première position d'extrémité (E1) en direction de la position intermédiaire (Z), une alarme puisse être émise du dispositif de commande et, lors d'un autre pivotement du moyen d'actionnement en direction de la seconde position d'extrémité (E2), le module (2) puisse être déconnecté par le dispositif de commande, dans lequel le moyen d'actionnement est formé par un disque (140) logé de manière rotative autour de l'axe de pivotement (141), qui présente deux évidements de mise en prise (143, 145) s'étendant radialement vers l'intérieur séparés l'un de l'autre par une nervure (144) pour un doigt et le moyen d'actionnement est agencé de manière adjacente à un emplacement d'enfichage de module (10) de sorte qu'un premier évidement de mise en prise (143) soit accessible de l'extérieur dans la première position d'extrémité (E1) et un pivotement soit permis au moyen d'une mise en prise de doigt dans le premier évidement de mise en prise (143) jusqu'à la position intermédiaire (Z), pendant que le second évidement de mise en prise (145) est recouvert et le second évidement de mise en prise (145) soit accessible de l'extérieur dans la position intermédiaire (Z) et un pivotement du disque (140) jusqu'à la seconde position d'extrémité (E2) soit permis au moyen d'une mise en prise de doigt dans le second évidement de mise en prise (145).

2. Appareil modulaire selon la revendication 1, **caractérisé en ce que** les dispositifs de verrouillage présentent un évidement de verrouillage (13) dans la zone des emplacements d'enfichage de module (10) de l'appareil de base (1) et les modules (2) présentent un crochet (21) en saillie introductible dans l'évidement de verrouillage (13) et le moyen d'actionnement est formé de sorte qu'il vienne en prise verrouillante à l'extérieur de la seconde position d'extrémité (E2) derrière le crochet (21) en saillie du module (2) dans l'évidement de verrouillage (13).

3. Appareil modulaire selon l'une quelconque des revendications 1 et 2, **caractérisé en ce que** les moyens de détection comprennent des capteurs magnétiques.

4. Appareil modulaire selon la revendication 3, **caractérisé en ce que** des capteurs à effet Hall (146, 147) sont prévus en tant que moyens de détection magnétiques et le moyen d'actionnement présente plusieurs éléments magnétiques (148, 149), qui peuvent être détectés au moyen des capteurs à effet Hall (146, 147).

5. Appareil modulaire selon la revendication 4, **caractérisé en ce qu'**un drapeau de commutation (142), qui porte les éléments magnétiques (148, 149), est prévu associé au moyen d'actionnement et pivotant conjointement avec celui-ci autour de l'axe de pivotement (141).

6. Appareil modulaire selon l'une quelconque des revendications 1 ou 2, **caractérisé en ce que** les moyens de détection comprennent des capteurs optiques.

7. Appareil modulaire selon l'une quelconque des revendications 1 à 6, **caractérisé en ce que** le moyen d'actionnement dans la première et la seconde position d'extrémité (E1, E2) ainsi que dans la position intermédiaire (Z) s'enclenche respectivement dans celle-ci de manière amovible et est précontraint entre la première position d'extrémité (E1) et la position intermédiaire (Z) en direction de la première position d'extrémité (E1) et est précontraint entre la position intermédiaire (Z) et la seconde position d'extrémité (E2) en direction de la position intermédiaire (Z).

8. Appareil modulaire selon l'une quelconque des revendications 1 à 7, **caractérisé en ce qu'**un logiciel fonctionnant sur le module (2) peut être arrêté au moyen du dispositif de commande avant la déconnexion d'un module (2).
